# EUROPEAN PATENT SPECIFICATION

(11) **EP 4 193 902 B1**
(45) Date of publication and mention of the grant of the patent: **02.04.2025**
(21) Application number: 22209373.4
(22) Date of filing: 24.11.2022
(51) Int. Cl.: A61B 1/00, A61B 1/018, A61B 1/307, A61B 1/31

(54) **HAND-HELD SURGICAL DEVICE AND SHAFT FOR A HAND-HELD SURGICAL DEVICE**
CHIRURGISCHE HANDVORRICHTUNG UND SCHAFT FÜR EINE CHIRURGISCHE HANDVORRICHTUNG
DISPOSITIF CHIRURGICAL PORTATIF ET TIGE POUR UN DISPOSITIF CHIRURGICAL PORTATIF

(30) Priority: 13.12.2021 US 202163288866 P
(43) Date of publication of application: 14.06.2023
(73) Proprietor: Olympus Winter & Ibe GmbH, 22045 Hamburg (DE)
(72) Inventor: Schmuck, Irina, 25746 Heide (DE); Schulz, Kevin Alexander, 22889 Wilstedt (DE); Schröder, Bastian, 22589 Hamburg (DE); Rühs, Andreas, 22926 Ahrensburg (DE)
(74) Representative: Hoener, Matthias

(56) References cited:
- EP-A1- 2 859 835
- WO-A1-2005/048827
- GB-A- 1 078 036
- US-A- 4 132 227
- US-A1- 2008 214 895
- US-A1- 2010 198 012
- US-A1- 2017 319 233

## Description

The invention relates to a shaft for a hand-held surgical device according to claim 1. The invention further relates to a hand-held surgical device according to claim 7.

US 2008/214895 A1 discloses a shaft for a hand-held surgical device according to the preamble of claim 1. US 2010/198012 A1, EP 2 859 835 A1, US 2017/319233 A1, WO 2005/048827 A1, US 4 132 227 A, and GB 1 078 036 A disclose similar devices.

Hand-held surgical devices, such as endoscopes, resectoscopes, cystoscopes and the like, are essentially constructed from a device or main body and a tube-like shaft. The tubular shaft may be, for example, an outer shaft. Inside the outer shaft, various other tube-like shafts, for example inner shafts, may be mounted parallel to the longitudinal direction of the outer shaft. The inner shafts may contain various instruments for medical treatment, e.g. an optic or, in the case of a resectoscope, an electrode carrier. It is intended for most instruments to be movable in the outer shaft parallel to the longitudinal direction of the outer shaft. In addition to the optics, other instruments can be inserted into the shaft. The space in the shaft that is not occupied by the optics is called the working channel. The other instruments can be inserted into the shaft in this working channel. In addition to the outer circumference of the shaft, the size or diameter of the working channel is an important parameter of the surgical handpiece.

Handheld surgical devices of the type described here are mainly used for endoscopic applications in the human body. For this purpose, the tube-like shaft of a hand-held surgical device is inserted into an opening of the human body. In the process, the distal end of the outer shaft and possibly also the distal ends of inner shafts come into direct contact with the human tissue. This contact generally involves a risk of injury to the human tissue. The risk of injury comes in particular from the edge of the distal end of the tube-like shaft. For example, cystoscopes are used for diagnostic or therapeutic procedures in the lower urinary tract. In this case, the patient's urethra is not arbitrarily stretchable and thus limits the circumference of the shaft of the cystoscope that can be inserted into the patient. In order not to injure the patient, the shaft should not be larger than is anatomically possible. To the extent that a shaft with a larger circumference cannot be used, this also means that certain instruments may not be able to be used to treat the patient, as the working channel would be too small. Therefore, the aim is to use a shaft whose working channel has an optimal value in relation to the outer circumference of the shaft. In order to optimise the ratio of the outer circumference of the shaft to the diameter of the working channel, a profile shape of the shaft must first be selected that forms as few dead spaces as possible within the shaft. In addition, all manufacturing tolerances must be reduced to ensure a minimum passage. Another possibility to reduce the dimension of the shaft is to reduce the wall thickness of the shaft and to avoid constrictions within the shaft. However, shafts with such reduced outer circumference must not have sharp edges or rims at the ends, especially at the distal end. For this purpose, it is known that edges of the shaft have a thickening in order to avoid the risk of traumatising the patient. Such thickenings, however, lead to the fact that the inner circumference of the shaft is reduced. This also reduces the volume of the working channel for receiving instruments.

The present invention is based on the task of creating a shaft and a hand-held surgical device which has an optimised shape for receiving an instrument.

A solution to this problem is described by the measures of claim 1. Accordingly, it is provided that a circumferential edge of a distal opening of a shaft for a hand-held surgical device has, at least in sections, an outwardly facing thickening, in particular a material thickening. Due to the rounded edges of the distal opening, this thickening can prevent cuts to the patient. The outward displacement of the thickening frees up the inner space or the working channel of the shaft for the insertion of an instrument. The size of the working channel is thus optimised or maximised. Due to the claimed sectional enlargement of the outer circumference of the shaft, the insertion of the hand device or shaft into the human body can also be improved. It proves to be particularly advantageous compared to an instrument whose outer diameter is enlarged over the entire length of the shaft.

In particular, the invention provides that the entire circumferential edge of the distal opening has the outwardly facing thickening. Due to this continuous thickening of the edge, traumatisation of the patient can be virtually excluded. In addition, the thickening can increase the stability of the shaft, especially in the distal area.

The invention provides for the distal opening to be oblique. This sloping course or sloping formation of the opening reduces the outer circumference of the shaft towards the distal end. This reduction of the outer circumference can be continuous or gradual, depending on the shape of the bevel. In this regard, an essential feature of the invention is that the outer circumference or diameter of the shaft decreases despite the thickening of the circumferential edge of the bevelled distal end. Only in the most proximal region of the shaft, where the bevelling begins, is the outer diameter of the shaft increased relative to the rest of the shaft due to the thickening. It is further conceivable that the outer circumference of the shaft is only increased over a length of the shank of 0.5 mm to 5 mm, in particular of 1 mm, due to the thickening of the edge. Furthermore, it is conceivable that the outer circumference of the shaft is only increased by the thickening of the edge over a length of the shaft of 0.1 % to 1.0 %, in particular 0.5 %. These values have proven to be particularly advantageous for their application.

The invention provides that the edge of the distal opening comprises two oblique portions and a horizontal portion oriented at least substantially parallel to a longitudinal axis of the shaft. Such a stepped shape of the distal opening is particularly suitable for insertion of the device into the human body as well as for use of the optic in connection with another device to be passed through the working channel of the shaft.

According to the invention, the thickness of the thickening of the opening changes continuously along the tube. This continuous change in the dimensioning of the thickening can be realised by an optimised shape of the opening for insertion into the body as well as the use of further instruments within the shaft. Furthermore, by adjusting the thickening of the opening, the stability of the shaft can be maximised while reducing the material.

A particularly advantageous embodiment of the invention may provide that the thickening has a round, preferably a circular, cross-section. Such shapes are optimally adapted to the anatomical conditions. However, it may also prove advantageous if the thickening has a different shape, for example to increase stability or to save material.

A hand-held surgical device for solving the above-mentioned problem is described by the features of claim 7.

Accordingly, it is provided that a hand-held surgical instrument, in particular a cystoscope, a resectoscope or the like, has a shaft according to at least one of claims 1-6.

A preferred embodiment of the invention is described in more detail below with reference to the drawing. In this show:
- Fig. 1a: schematic representation of a hand-held surgical device,
- Fig. 2: a schematic view of a distal end of the surgical device,
- Fig. 3: a schematic representation of a distal end portion of a tubular shaft, and
- Fig. 4: a schematic representation of an area of the distal end section.

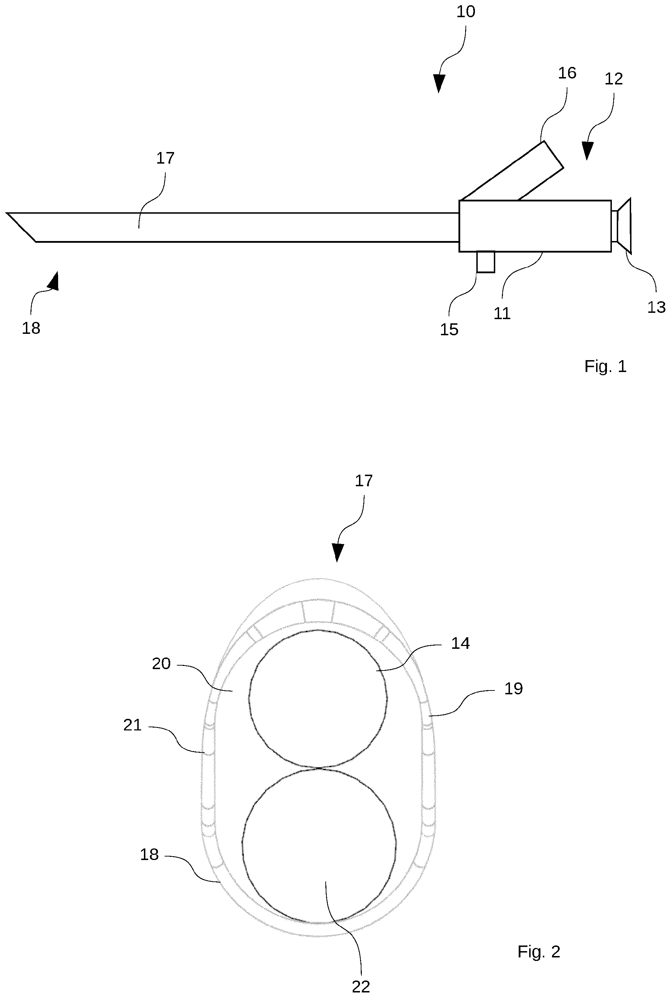

In Fig. 1a hand-held surgical device 10 is shown in a strongly schematised and symbolised form for better illustration. This surgical device 10 has a instrument body 11, which can also be designed as a handle unit. Furthermore, an ocular 13 for an optical system 14 is arranged at a proximal end 12 of the instrument body 11. In addition, the instrument body 11, shown here purely schematically, has two connections 15, 16. These connections 15, 16 can be used to supply the surgical device 10 with electrical energy, light, a fluid or as a port for further instruments.

A tube-like shaft 17 is arranged at an end opposite the proximal end 12 of the instrument body 11. This shaft 17 is elongated and serves to be inserted into the patient with a distal end 18. This shaft 17 is also shown in Fig. 1 in a highly schematised form and serves only to provide a better understanding of the invention described herein. It should be expressly noted that such a shaft 17 can also be coupled with other surgical devices. Thus, the oblique design of the distal end 18 of the shaft 17 is also to be regarded as highly schematised. In fact, it is quite envisaged that the shape of the bevel can deviate greatly from that shown in Fig. 1.

The invention provides that a circumferential edge 19 of a distal opening 20 of the shaft 17 has a thickening 21 at least in sections. This thickening 21 of the circumferential edge 19 is shown in Fig. 2. This Fig. 2 shows a view of the distal opening 20 of the shaft 17. In the embodiment example shown here, it can be seen that the thickening 21 extends over the entire circumferential edge 19. This thickening 21 increases the outer circumference or the diameter of the shaft 17 in certain areas. Since this thickening 21 extends outwards, the inner circumference remains unaffected by this thickening 21, i.e. the full inner volume of the shaft 17 is available for inserting or passing through instruments.

It can be seen from Fig. 2 that in the embodiment example shown here, a highly schematised optic 14, which can be designed as a light guide or rod lens system, is arranged within the shaft 17. The space around the optics 14 or below the optics 14 is referred to as the working space. Through this working space, further instruments can be introduced through the shaft 17 into the interior of the patient to be treated. In the embodiment example shown here, this working space is marked as a circle 22. By moving the thickening 21 from the interior of the shaft 17 to the outside, the optics 14 and the working space have the largest possible volume at their disposal.

As already explained above, this thickening 21 of the circumferential edge 19 is imperative in order not to traumatise the patient with the sharp edges of the distal end 18 of the shaft 17. The thickening 21 can have a circular, oval or similar cross-section with a diameter of a few tenths of a millimetre.

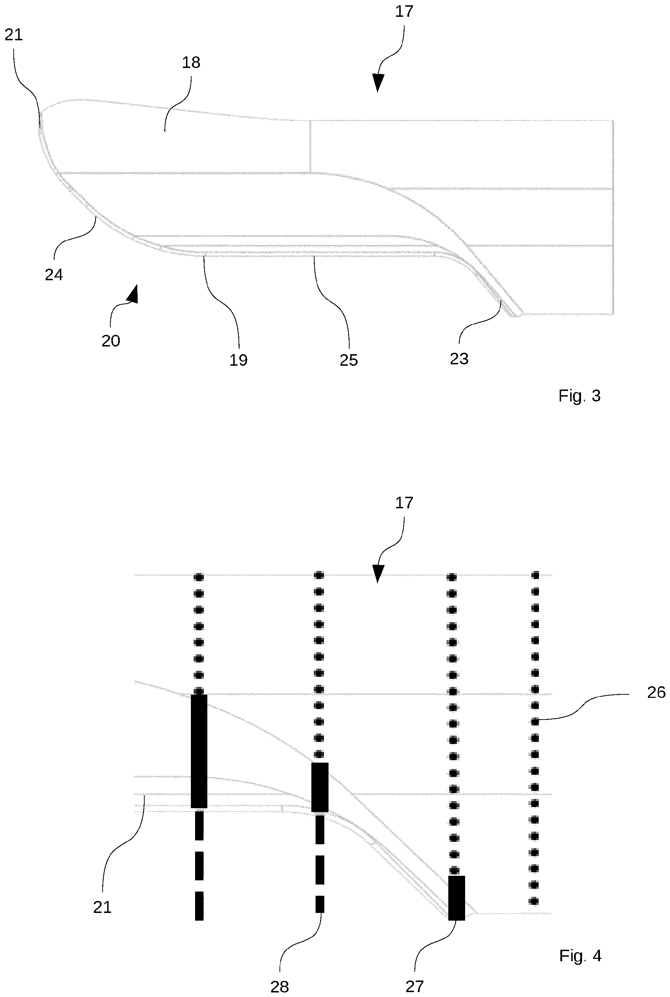

In Fig. 3 the distal end 18 of the shaft 17 is shown from the side. It can be seen that the distal opening 20 is bevelled. The shape of this bevel is beak-like. While the opening 20 has two sloping portions 23, 24, a horizontal section extends between these two portions 23, 24. It has been shown that this shape of the distal opening 20 is particularly well suited for inserting the shaft 17 into the patient in a way that is gentle on the body. In particular, due to the thickening 21 of the circumferential edge 19, traumatisation or injuries can be avoided. At the same time, instruments such as an optic 14 can be efficiently guided through the shaft 17 by moving the thickening 21 outwards.

Due to the shape of the distal opening 20 of the shaft 17 shown here, the outer circumference of the shaft 17 is only enlarged over a very short distance by the thickening 21. This is illustrated in Fig. 4. Fig. 4 shows a section of the distal opening 20 as shown in Fig. 3. The dotted line 26 represents the unchanged area of the shaft 17, which corresponds to the diameter of the shaft 17. The solid line 27 indicates the area of the stem 17 whose outer circumference is increased by the thickening 21 compared to the rest of the shaft 17 (dotted line 26). The dotted line 28 marks the area of the shaft 17 where the outer circumference or diameter is reduced by the shape of the distal opening 20. Because the thickening 21 follows the sloping distal opening 20, the outer circumference of the largest cross-section is only slightly increased. Only about 1 mm before the beginning of the distal opening 20 is the outer circumference slightly increased. As the distal opening 20 bevels, the outer circumference of the shaft 17 decreases despite the thickening 21. It has been shown that the shaft 17 with a very locally increased outer circumference can be inserted into the patient better than a shaft which has a correspondingly increased outer circumference over the entire length of the shaft.

### List of reference signs:

- 10: hand-held surgical device
- 11: instrument body
- 12: proximal end
- 13: ocular
- 14: optics
- 15: connection
- 16: connection
- 17: shaft
- 18: distal end
- 19: circumferential edge
- 20: distal opening
- 21: thickening
- 22: circle
- 23: oblique portions
- 24: oblique portions
- 25: horizontal portions
- 26: dotted line
- 27: continuous line
- 28: dotted line

## Claims

1. A shaft (17) for a hand-held surgical device (10), in particular for a cystoscope, for receiving an optical system (14) and at least one further instrument, wherein a proximal end of the tubular shaft (17) can be coupled to an instrument body (11) and/or a handle unit and wherein the optical system (14) and the instrument can be positioned with their distal ends inside a distal opening (20), preferably a window opening, of the shaft (17), wherein a circumferential edge (19) of the distal opening (20) of the shaft (17) has, at least in sections, an outwardly facing thickened portion (21), wherein the distal opening (20) is formed obliquely, **characterized in that** the edge (19) of the distal opening (20) comprises two oblique portions (23, 24) and a horizontal portion (25) oriented at least substantially parallel to a longitudinal axis of the shaft (17), and that the thickness of the outwardly facing thickened portion (21) of the opening (20) varies continuously along the shaft (17).

2. A shaft (17) for a hand-held surgical device (10) according to claim 1, **characterised in that** the entire circumferential edge (19) of the distal opening (20) has the outwardly facing thickened portion (21).

3. A shaft (17) for a hand-held surgical device (10) according to claim 1, **characterised in that** an outer circumference of the shaft (17) is enlarged only in regions by the outwardly facing thickened portion (21) of the edge (19).

4. A shaft (17) for a hand-held surgical device (10) according to claim 3, **characterised in that** the outer circumference of the shaft (17) is enlarged by the outwardly facing thickened portion (21) of the edge (19) only over a length of the shaft (17) of 0.5 mm to 5 mm, in particular of 1 mm.

5. A shaft (17) for a hand-held surgical device (10) according to claim 3, **characterised in that** the outer circumference of the shaft (17) is increased by the outwardly facing thickened portion (21) of the edge (19) only over a length of the shaft (17) of 0.1% to 1.0%, in particular 0.5%.

6. A shaft (17) for a hand-held surgical device (10) according to any one of the preceding claims, **characterised in that** the outwardly facing thickened portion (21) has a round, preferably a circular, cross-section.

7. Hand-held surgical device (10), in particular a cystoscope, resectoscope or the like, having a shaft (17) according to at least one of claims 1 to 6.

## Patentansprüche

1. Schaft (17) für eine handgeführte chirurgische Vorrichtung (10), insbesondere für ein Zystoskop, zur Aufnahme eines optischen Systems (14) und mindestens eines weiteren Instruments, wobei ein proximales Ende des rohrförmigen Schafts (17) an einen Instrumentenkörper (11) und/oder eine Griffeinheit gekoppelt werden kann, und wobei das optische System (14) und das Instrument mit ihren distalen Enden innerhalb einer distalen Öffnung (20), vorzugsweise einer Fensteröffnung, des Schafts (17) positioniert werden können, wobei ein Umfangsrand (19) der distalen Öffnung (20) des Schafts (17) zumindest abschnittsweise einen auswärts weisenden verdickten Anteil (21) aufweist, wobei die distale Öffnung (20) schräg ausgebildet ist, **dadurch gekennzeichnet, dass** der Rand (19) der distalen Öffnung (20) zwei schräge Anteile (23, 24) und einen horizontalen Anteil (25) umfasst, der zumindest im Wesentlichen parallel zu einer Längsachse des Schafts (17) orientiert ist, und dass die Dicke des auswärts weisenden verdickten Anteils (21) der Öffnung (20) entlang des Schafts (17) kontinuierlich variiert.

2. Schaft (17) für eine handgeführte chirurgische Vorrichtung (10) nach Anspruch 1, **dadurch gekennzeichnet, dass** der gesamte Umfangsrand (19) der distalen Öffnung (20) den auswärts weisenden verdickten Anteil (21) aufweist.

3. Schaft (17) für eine handgeführte chirurgische Vorrichtung (10) nach Anspruch 1, **dadurch gekennzeichnet, dass** ein Außenumfang des Schafts (17) nur in Regionen durch den auswärts weisenden verdickten Anteil (21) des Randes (19) vergrößert ist.

4. Schaft (17) für eine handgeführte chirurgische Vorrichtung (10) nach Anspruch 3, **dadurch gekennzeichnet, dass** der Außenumfang des Schafts (17) durch den auswärts weisenden verdickten Anteil (21) des Randes (19) nur über eine Länge des Schafts (17) von 0,5 mm bis 5 mm, insbesondere von 1 mm, vergrößert ist.

5. Schaft (17) für eine handgeführte chirurgische Vorrichtung (10) nach Anspruch 3, **dadurch gekennzeichnet, dass** der Außenumfang des Schafts (17) um den auswärts weisenden verdickten Anteil (21) des Randes (19) nur über eine Länge des Schafts (17) von 0,1 % bis 1,0 %, insbesondere 0,5 % vergrößert ist.

6. Schaft (17) für eine handgeführte chirurgische Vorrichtung (10) nach einem der vorhergehenden Ansprüche, **dadurch gekennzeichnet, dass** der auswärts weisende verdickte Anteil (21) einen runden, vorzugsweise kreisförmigen Querschnitt aufweist.

7. Handgeführte chirurgische Vorrichtung (10), insbesondere ein Zystoskop, Resektoskop oder dergleichen, mit einem Schaft (17) nach mindestens einem der Ansprüche 1 bis 6.

## Revendications

1. Tige (17) pour un dispositif chirurgical manuel (10), en particulier pour un cystoscope, destinée à recevoir un système optique (14) et au moins un autre instrument, une extrémité proximale de la tige tubulaire (17) pouvant être couplée à un corps d'instrument (11) et/ou à une unité de poignée et le système optique (14) et l'instrument pouvant être positionnés avec leurs extrémités distales à l'intérieur d'une ouverture distale (20), de préférence une ouverture de fenêtre, de la tige (17), un bord circonférentiel (19) de l'ouverture distale (20) de la tige (17) ayant, au moins en sections, une partie épaissie tournée vers l'extérieur (21), l'ouverture distale (20) étant formée obliquement, **caractérisée en ce que** le bord (19) de l'ouverture distale (20) comprend deux parties obliques (23, 24) et une partie horizontale (25) orientées au moins sensiblement parallèlement à un axe longitudinal de la tige (17), et **en ce que** l'épaisseur de la partie épaissie tournée vers l'extérieur (21) de l'ouverture (20) varie de façon continue le long de la tige (17).

2. Tige (17) pour un dispositif chirurgical manuel (10) selon la revendication 1, **caractérisée en ce que** la totalité du bord circonférentiel (19) de l'ouverture distale (20) présente la partie épaissie tournée vers l'extérieur (21).

3. Tige (17) pour un dispositif chirurgical manuel (10) selon la revendication 1, **caractérisée en ce qu'**une circonférence extérieure de la tige (17) n'est élargie que dans des régions par la partie épaissie tournée vers l'extérieur (21) du bord (19).

4. Tige (17) pour un dispositif chirurgical manuel (10) selon la revendication 3, **caractérisée en ce que** la circonférence extérieure de la tige (17) est élargie par la partie épaissie tournée vers l'extérieur (21) du bord (19) uniquement sur une longueur de la tige (17) de 0,5 mm à 5 mm, en particulier de 1 mm.

5. Tige (17) pour un dispositif chirurgical manuel (10) selon la revendication 3, **caractérisée en ce que** la circonférence extérieure de la tige (17) est augmentée par la partie épaissie tournée vers l'extérieur (21) du bord (19) uniquement sur une longueur de la tige (17) de 0,1 % à 1,0 %, en particulier 0,5 %.

6. Tige (17) pour un dispositif chirurgical manuel (10) selon l'une quelconque des revendications précédentes, **caractérisée en ce que** la partie épaissie tournée vers l'extérieur (21) a une section transversale ronde, de préférence circulaire.

7. Dispositif chirurgical manuel (10), notamment cystoscope, résectoscope ou similaire, ayant une tige (17) selon au moins l'une des revendications 1 à 6.
